Europäisches Patentamt

European Patent Office

Office européen des brevets

(19)

(11) EP 1 066 119 B1

(12) EUROPEAN PATENT SPECIFICATION

(45) Date of publication and mention
of the grant of the patent:
**24.11.2004 Bulletin 2004/48**

(21) Application number: **99914182.3**

(22) Date of filing: **26.03.1999**

(51) Int Cl.[7]: **B06B 1/06**

(86) International application number:
**PCT/US1999/006650**

(87) International publication number:
**WO 1999/048621 (30.09.1999 Gazette 1999/39)**

(54) **ARRAYS MADE FROM FLEXIBLE TRANSDUCER ELEMENTS**

GRUPPIERUNGEN VON BIEGSAMEN WANDLERELEMENTEN

MATRICES D'ELEMENTS TRANSDUCTEURS SOUPLES

(84) Designated Contracting States:
**AT BE CH CY DE DK ES FI FR GB GR IE IT LI LU MC NL PT SE**

(30) Priority: **26.03.1998 US 79428 P**

(43) Date of publication of application:
**10.01.2001 Bulletin 2001/02**

(60) Divisional application:
**04016458.4**

(73) Proprietors:
• **Exogen, Inc.**
**Memphis, Tennessee 38116 (US)**
• **RUTGERS, THE STATE UNIVERSITY OF NEW JERSEY**
**Piscataway, NJ 08854-8010 (US)**

(72) Inventors:
• **CORNEJO, Ivan A.**
**Highland Park, NJ 08904 (US)**
• **JADIDIAN, Bahram**
**Dayton, NJ 08810 (US)**
• **SAFARI, A.**
**Princeton Junction, NJ 08550 (US)**
• **WINDER, Alan, A.**
**Westport, CT 06880 (US)**

(74) Representative: **Laufhütte, Dieter, Dr.-Ing. et al**
**Lorenz-Seidler-Gossel**
**Widenmayerstrasse 23**
**80538 München (DE)**

(56) References cited:
**WO-A-94/13411          WO-A-95/03744**
**US-A- 4 726 099**

• **PATENT ABSTRACTS OF JAPAN vol. 013, no. 541 (E-854), 5 December 1989 (1989-12-05) -& JP 01 223785 A (OKI ELECTRIC IND CO LTD), 6 September 1989 (1989-09-06)**

**EP 1 066 119 B1**

**Description**

BACKGROUND OF THE INVENTION

1. Technical Field

Novel flexible piezoelectric transducer elements and large-area flexible arrays of such transducers are described herein. Also described are methods for manufacture of the flexible transducer elements and transducer arrays. Additionally, use of the transducer elements or transducer arrays in medical therapeutic ultrasound applications, e.g., for promoting the healing of bone fractures and tissue wounds, medical diagnostic ultrasound applications and nondestructive testing are also described herein.

2. Background of Related Art

The use of ultrasound to therapeutically treat and evaluate bone fractures is known. Impinging ultrasonic pulses having appropriate dosage parameters, e.g., frequency, acoustic intensity, duty cycle, pulse repetition rate and total treatment time, and administered externally at the fracture site has been determined to accelerate the natural healing of, for example, bone fractures. For patients with reduced healing capacity, such as elderly persons with osteoporosis, ultrasonic therapy may promote healing of bone injuries that would otherwise require prosthetic replacement or leave the patient permanently disabled.

U.S. Patent No. 4,530,360 to Duarte describes a basic non-invasive therapeutic technique and apparatus for applying ultrasonic pulses externally on the skin of the patient at a location adjacent to the bone fracture site. The applicator described in the '360 patent has a plastic tube which serves as a grip for the operator, an RF plug attached to the plastic tube for connection to an RF source, and internal cabling connected to a rigid ultrasonic transducer. To apply the ultrasound pulses during treatment, an operator manually holds the applicator in place until the treatment is complete. The '360 patent also describes a range of RF signals for creating the ultrasound, ultrasound power density levels, a range of duration for each ultrasonic pulse, and a range of ultrasonic pulse frequencies. U.S. Patent Nos. 5,003,695; 5,211,160 and 5,762,616 relate to ultrasonic body treatment systems that include rigid transducer elements and structure for fixing the transducer adjacent body tissue.

While these prior art systems provide accelerated healing of soft tissue wounds and bone fractures, the transducer elements and the arrays of such elements are too rigid to conform to large area contours of the body such as, for example, the contours of the hip and spine. It would be very desirable to provide flexible transducer elements and flexible, unitary arrays containing such flexible transducer elements that conform to body contours to facilitate the effective application of therapeutic ultrasonic dosages.

Previous attempts have been made to provide flexible transducer elements.

Patent Applicaton WO 94/13411 discloses an ultrasonic transducer comprising a flexible transmitter, a flexible receiver array and flexible electrodes for the transmitter and the receiver. The transmitter comprises an array of piezoceramic rods embedded within a polymer matrix. In another embodiment piezoceramic platelets are embedded in a flexible polymer substrate of the flexible transmitter.

For example, U.S. Patent No. 4,227,111 discloses a flexible, low density, piezoelectric composite transducer in which the piezoelectric composite is formed by cojoining a polymer phase and a piezoelectric phase in such a manner that each individual phase is interconnected in all three orthogonal dimensions. As another example, U.S. Patent No. 4,233,477 discloses a flexible, composite, acoustical energy transducer made from ferroelectric, piezoelectric and/or electrostrictive materials arranged in the same x-y plane within a polymer having electroelastic properties. The composite is flexible so it can be made into different shapes such as flat, bent, and cylindrical and/or secured to a curved or differently shaped body. Yet another example is U.S. Patent No. 5,691,960 which discloses acoustic transducer panels fabricated as a composite of a piezoelectric or electrostrictive ceramic material and a polymeric material.

A common deficiency of flexible transducer elements is a lack of physical integrity. Due to poor adhesion between ceramic and polymeric components, physical separation of the components may occur upon bending of the transducer element. Physical separation of the components can introduce air pockets that interfere with operation of the transducer element. Maintenance of physical integrity is especially troublesome where the transducer element is intended for therapeutic applications and includes a matching layer or where a monolithic array of such transducer elements is flexed.

It would be desirable to provide a flexible piezoelectric ceramic/polymer composite transducer element or a flexible, unitary transducer array of such flexible transducer elements that can be conformed to the contours of the human anatomy in need of therapeutic stimulation.

## SUMMARY OF THE INVENTION

Novel flexible piezoelectric composite transducer elements and large-area flexible arrays of such transducer elements for use in therapeutic applications have been discovered. The novel transducer arrays include at least two flexible composite transducer elements arranged in adjacent relation with polymer-filled spaces therebetween, electrode surfaces applied to opposite surfaces of the array and a matching layer applied to, and substantially coextensive with, one of the electrode surfaces.

In one embodiment, composite transducer elements used to make the arrays are obtained by dicing and filling a piezoelectric wafer. That is, the composite transducer elements are obtained by forming a first and second set of channels in the piezoelectric material, forming a film of polymeric material on the piezoelectric material and covering the film with a second polymeric material in an amount sufficient to fill the first and second set of channels.

Methods for making large-area flexible piezoelectric composite transducer arrays have also been discovered. In the method, a plurality of the flexible composite transducer elements are arranged in adjacent relation to define spaces therebetween to form an array, the spaces are filled with a polymeric material, electrodes are applied to opposite surfaces of the array and a matching layer is applied to one of the electrode surfaces.

A method for using the piezoelectric composite transducer elements and the flexible transducer arrays in therapeutic applications has also been discovered. In the method, flexible transducer elements or flexible transducer arrays of the transducer elements can be applied to a section of the human anatomy in need of therapeutic stimulation and that section of the human anatomy is then exposed to a dosage amount of acoustic energy.

The transducer elements and large-area flexible transducer arrays described herein possess sufficient flexibility such that the transducers advantageously permit acoustic energy generated by the transducer to be efficiently applied and coupled to the contours of the human anatomy for therapeutic and diagnostic applications.

## BRIEF DESCRIPTION OF THE DRAWINGS

Preferred embodiments of the invention are described below with reference to the drawing, which are described as follows:

FIG. 1A is a schematic view of a piezoelectric material having interconnecting channels formed therein to be used in forming one type of composite transducer element in accordance with the present invention;
FIG. 1B is a cross-sectional schematic view of the material of FIG. 1A with a matching layer formed thereon;
FIG. 1C is a cross-sectional schematic view of the material of FIG. 1B with an electrode surface applied to opposite surfaces of the material and a matching layer formed on one surface thereof to form a composite transducer element;
FIG. 2 is a cross-sectional schematic view of an array of the composite transducer elements of FIG. 1C used to form a flexible transducer array in accordance with the present invention.

## DESCRIPTION OF THE PREFERRED EMBODIMENTS

A flexible piezoelectric composite transducer array in accordance with this disclosure includes an array containing a plurality of piezoelectric composite transducer elements arranged in adjacent relation to define spaces therebetween with a polymeric material disposed in the spaces, electrodes applied to opposite surfaces of the array and a matching layer applied to, and substantially coextensive with, one of the electrode surfaces. The composite transducer elements which make up the transducer array are themselves flexible, e.g., have a flexibility of at least 2.0 mm as measured by a four-point bend test. The novel transducer arrays made from these flexible transducer elements are likewise flexible and can conform to different contours of the human anatomy when employed for therapeutic application.

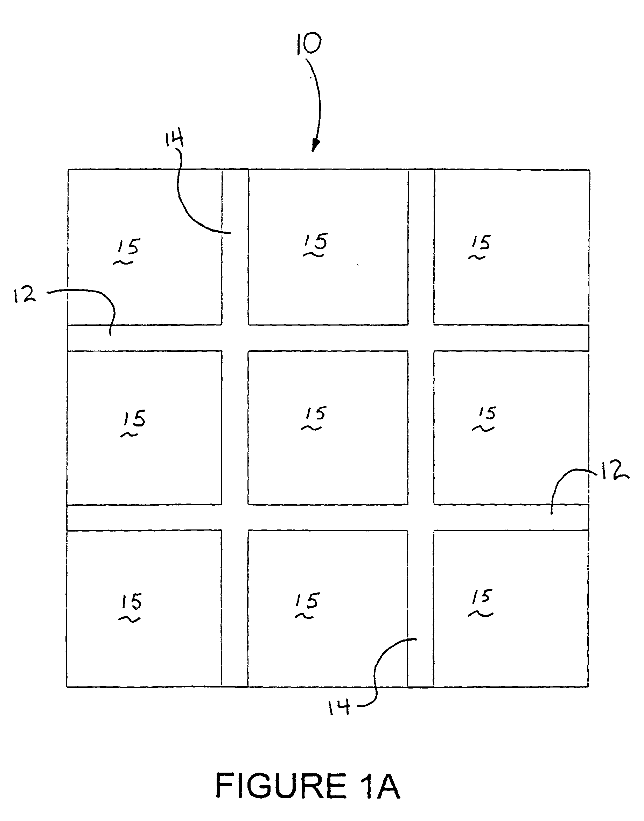

As seen in the embodiments shown in FIG. 1A-1C, one type of composite transducer element for use herein can be formed by first providing a piezoelectric material 10. Suitable piezoelectric materials for this composite include PZT powders commercially available from Morgan Matroc, Inc., ceramic, single-crystal relaxor ferroelectric, lead zirconate titanate $Pb(Zr,Ti)O_3$, lead metaniobate $Pb(Nb_2O_6)$, modified lead titanate $PbTi_3$ such as $(Pb,Ca)TiO_3$ and $(Pb,Sm)TiO_3$, barium titanate $BaTiO_3$, PMN-PT$(1-x)Pb(Mg_{1/3}Nb_{2/3})O_3-xPbTiO_3$, PZN-PT/BT $Pb(Zn_{1/3}Nb_{2/3})O_3-xPbTiO_3-BaTiO_3$, $(1-x)Pb(Zn_{1/3}, Nb_{2/3})O_3-x(yPbTiO_3-(1-y)PbZrO_3)$ and the like. In general, the piezoelectric material 10 is processed by first pressing the material uniaxially at a pressure from about $1,034.10^8$ Pa (15 ksi) to about $3,447.10^8$ Pa (50 ksi) and then sintering the pressure material at a temperature ranging from about 1000°C to about 1400°C and preferably from about 1150°C to about 1300°C for a time period not exceeding about 2 hours. A lead source, e.g., a mixture of PbO and $ZrO_2$, can be added to piezoelectric material 10 when sintering to compensate for any loss of lead from the material 10.

Following processing, piezoelectric material 10 is subjected to dicing. Initially, a first set of channels 12 are formed parallel to each other and within material 10 (see FIG. 1A). Techniques for forming channels 12 are within the purview of one skilled in the art. Typically, the width of each channel 12 will range from about 25 to about 1,200 microns and preferably from about 200 to about 600 microns. Preferably, when forming the channel in parallel juxtaposition, the distance between the channels will range from about 200 to about 400 microns.

A second set of channels 14 is then be formed parallel to one another and angularly oriented with respect to each channel 12. In general, the sets of interconnecting channels can intersect at an angle of between about 5° and about 90° and preferably between about 30° and about 90°. It should be understood that an angle other than 90° between the channels may impart preferential bending in a desired plane. Therefore, perpendicular sets of channels are preferred. Thus, as generally depicted in FIG. 1A, each of channels 12 and 14 are linear and intersect to define islands 15 of piezoelectric material. Techniques for forming channels 14 are within the purview of one skilled in the art. Typically, the width of each channel 14 can be the same or different as each of channel 12. Thus, the width of channel 14 will ordinarily range from about 25 to about 1,200 microns and preferably from about 200 to about 600 microns. Preferably, when forming the channels in parallel juxtaposition, the distance between the channels will range from about 200 to about 400 microns. Preferably, the width and thickness of the channels is chosen so that the composite transducer element will contain from about 25 to 35 volume percent piezoelectric material with the remaining volume being substantially polymeric material.

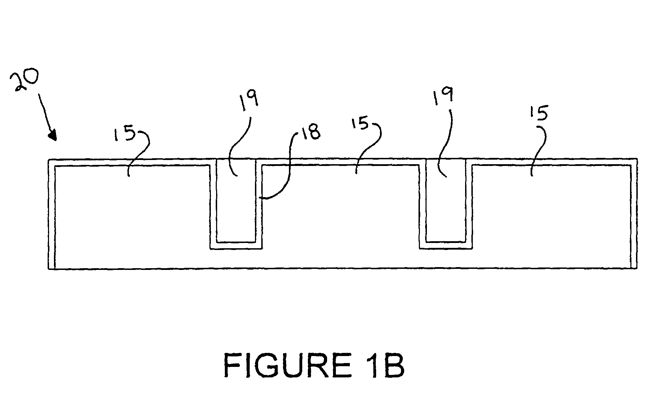

Following the formation of the first and second set of channels (i.e., dicing), a film 18 of a polymeric material is formed on the piezoelectric material and within the channels as shown in FIG. 1B. In general, film 18 is formed by filling the channels with a polymeric material before curing, excess polymer is removed by, for example, pressurized airing, and then followed by curing. As one skilled in the art can readily appreciate, film 18 can also be formed by other methods known to one skilled in the art, e.g., dip coating material 10 in a polymeric material, removing any excess polymer and then followed by curing. This will leave only a film of the polymer on the piezoelectric material. Suitable polymeric materials for forming the film include low viscosity Spurr epoxies, silicone and silane additives such as those available from Dow Corning, e.g., water-based acrylics, solvent-based acrylics, water-based polyesters, solvent-based polyesters, water-based alkyds, solvent-based alkyds, solvent-based amides, solvent-based nitrocellulose, water-based polyurethanes, solvent-based polyurethanes, solvent-based epoxies, water-based vinyls, solvent-based vinyls, solvent-based phenolics and the like with the Spurr epoxies being preferred.

Next, additional polymeric material 19 is deposited over the film to fill each of channels 12 and 14 to form a flexible composite 20 as depicted in FIG. 1B. Suitable polymeric materials for use herein include thermoplastics, thermosets, rubbers and mixtures thereof. Useful thermoplastics include high density polyethylenes, polymethyl methacrylates, polypropylenes, polybutylene terephthalates, polycarbonates, polyurethanes such as CA 118 and CA 128 available from Morton Chemical and estane polyester, and the like. Useful thermosets include epoxies such as Spurr epoxy and Stycast 80, and the like. Useful rubbers include silicone rubbers such as dispersion 236 available from Dow Corning and RTV-141 available from Rhone-Poulenc, Inc. and the like. Preferred polymeric materials for use herein include Stycast 1365-65 available from Emerson and Gumming, Deway and Almay Chemical division (Canton, MA) and Spurr epoxy available from Ernest F. Fullam Inc. (Schenectady, NY).

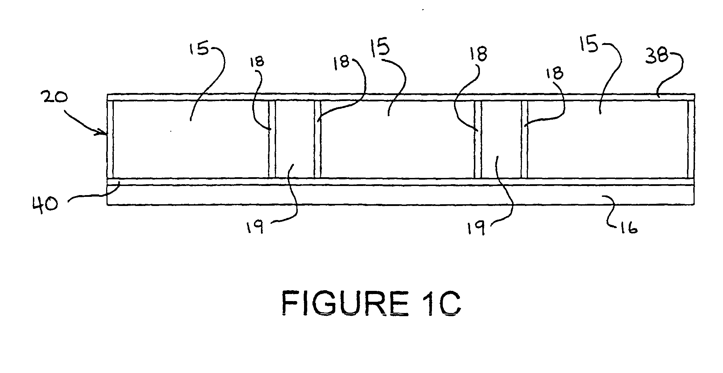

Following the depositing of polymeric material into each of channels 12 and 14, flexible composite 20 can be subjected to further processing to form the composite transducer element in accordance with the present invention. Further processing includes, for example, cutting composite 20 into slices and then polishing the surfaces thereof. Electrode surfaces 38 and 40 can then be applied to, and substantially coextensive with, opposite surfaces of the composite 20 (See FIG. 1C) by techniques known to one skilled in the art, e.g., sputtering, painting, etc. However, if the transducer elements are to be employed in an array to form a transducer array, the electrode surfaces may be applied at the time the array is formed as discussed below. Materials useful in forming electrode surfaces 38 and 40 include copper, silver, nickel, gold, alloys, mixtures thereof and the like.

Once electrode surfaces 38 and 40 have been applied to composite 20, a matching layer 16, which is discussed hereinbelow, can then be applied to, and substantially coextensive with, one of the electrode surfaces 38 and 40 (See Fig. 1C) to form the composite transducer element.

If the transducer elements are to be employed in an array to form a transducer may, the electrode surfaces may be applied at the time the array is formed as discussed below. Suitable materials for electrode surfaces include copper, silver, nickel, gold, alloy, mixtures thereof and the like. A matching layer, which is discussed hereinbelow, can then be applied to, and substantially coextensive with, one of the electroded surfaces to form the transducer element.

The composite transducer elements are tested for flexibility on an Instron device using the four point bend test described in ASTM method C 1161-90. The diced composites can withstand a 2mm bend without failure.

The flexible transducer array of the present invention can be obtained by forming an array of a plurality of the foregoing composite transducer elements. In general, the composite transducer elements can be placed close together or spaced further apart and the spacings need not be uniform or in perfect alignment. The composite transducer elements, however, are not initially secured to another. Thus, to form the array, the orientation of each composite transducer elements is determined and placed flat in, for example, a mold, in the desired arrangement. Typically, the space between the composite transducer elements will range from about 0.5mm to about 10mm and preferably from about 1mm to about 3mm. A polymeric material 36 is then back-filled into the spaces formed between the arranged composite transducer elements to adhere the elements together and form a coherent array. The dimensions of the array will typically be in the range of from about 1,27 cm (0.5 inch) to about 15,24 cm (6 inches) wide and from about 1,27 cm (0.5 inch) to about 30,48 cm (12 inches) long. The thickness of the array can effect the frequency of operation and will ordinarily range from about 0.05mm to about 10mm. It should, of course, be understood that the array can include elements with different frequencies of operation. These differences in frequency of operation can be achieved by employing composites of different thicknesses or having different levels of polymer loading. Suitable polymeric materials include those polymeric materials discussed above with the preferable polymeric material being Stycast 1365-65.

Once electrode surfaces have been applied, matching layer can then be applied to, and substantially coextensive with, an electrode surface. Techniques for applying the matching layer are within the purview of one skilled in the art. Generally, the thickness of the matching layer can be chosen to correspond to one-quarter of the wavelength in the matching layer at the operating frequency of the transducer array. The acoustic impedance of the matching layer will preferably range from about 2.0 to about 7.0 MRayls and more preferably from about 3.0 to about 4.0 MRayls.

Matching layer will ordinarily be formed from a polymeric material, and optionally, a filler. The polymeric material should have good compatibility with the components of the composite, biocompatibility and flexibility. Useful polymeric materials include thermoplastics such as high density polyethylenes, polymethyl methacrylates, polypropylenes, polybutylene terephthalates, polycarbonates, polyurethanes such as CA 118 and CA 128 available from Morton Chemical and estane polyester, and the like; thermosets such as epoxies such as Spurr epoxy and Stycast 80, and the like; and rubbers such as silicone rubbers such as dispersion 236 available from Dow Corning and RTV-141 available from Rhone-Poulenc, Inc. and the like. A preferred polymeric material for use herein is Stycast 1365-65. Because the acoustic impedance of many polymeric materials is less than the preferred range of 3.0 - 4.0 MRayls, it is necessary to increase the acoustic impedance of the polymer. Accordingly, one or more fillers can be incorporated therein. Suitable fillers include PZT, tungsten, alumina, silica glass, tungsten carbide, titanium, glass powder and the like with glass powder being preferred. The size of the filler particles should be in the range of about 0.1 to about 50 microns and preferably from about 0.5 to about 5 microns. The amount of filler employed will be that amount necessary to impart the desired acoustic impedance. Normally, from about 2 to about 50 percent filler by volume and preferably from about 5 to about 30 volume percent filler is employed.

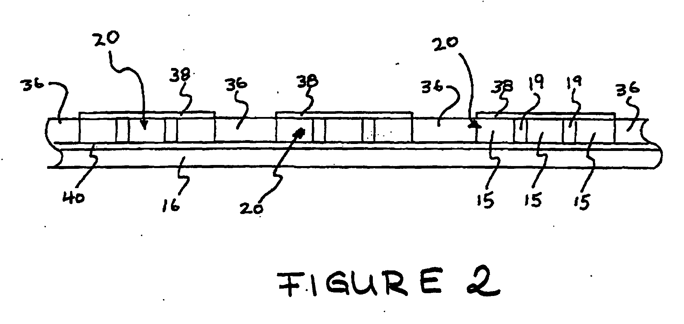

The large-area flexible transducer array can be obtained by forming an array of a plurality of the composite transducer elements formed from the aforementioned dicing and filling method. As depicted in FIG. 2 this transducer array will contain a plurality of the composite transducer elements 20 arranged in adjacent relation thereby forming spaces 36 which are back-filled with a polymeric material discussed above. Composite transducer element 20 will contain islands 15 of piezoelectric material having interconnecting channels which have a film formed thereon (not shown) and are then filled with a polymeric material 19. Next, electrode surfaces 38 are applied onto one surface of each composite transducer element 20 in the array employing techniques known in the art, e.g., photolithography techniques. Electrode surface 40 is then applied to, and substantially coextensive with, the other surface of the array. Then a matching layer 16 is applied to, and substantially coextensive with, electrode surface 40 as discussed above.

For convenience, a housing can be provided to at least partially surround the electroded flexible composite transducer element or array of such elements and any associated matching layer. Normally, the matching layer will not be covered by the housing to avoid undesired changes in the acoustic impedance of the outer most surface of the system. Alternatively, the matching layer can be integral with the housing. Any material can be used to form the housing. Preferably, biocompatible polymers are used. Where a flexible array of transducer elements is being produced, the housing material should be at least as flexible as the array. Polymeric materials are particularly suitable in this instance.

The transducer elements and transducer arrays of this invention are particularly useful in therapeutic applications. In general, the foregoing transducers will flex to conform to a part of the human anatomy, e.g., the hip, spine, etc., to facilitate the transfer of acoustic energy to promote healing of bone fractures and soft tissue wounds. Typically, the frequency of the acoustic energy will be in the range of from about 0.1 to about 10 MHz and preferably from about 0.5 to about 5 MHz. Features characterizing the frequency of the acoustic energy are disclosed in U.S. Patent No. 5,520,612, the contents of which are incorporated by reference herein. It is to be understood that multiple sections of the human anatomy can be treated with multiple transducer elements or transducer arrays at the same time. Thus, for example, in the case of an individual suffering from both a bone fracture in the hip and a soft tissue wound in the back, a transducer element or transducer array can be applied to the hip while a second transducer element or transducer array is being applied to the section of the back suffering from the wound. The transmit frequency and acoustic energy applied to each section can vary according to the foregoing ranges.

When applying the foregoing transducers to the section of the human anatomy in need of therapeutic assistance, it is advantageous to apply a coupling gel to the transducer prior to its employment on the part of the body. Additionally, when employing the foregoing transducer arrays, it may be desirable in certain cases to use selective energization of the transducer elements in the array such that certain elements will generate acoustic energy while other elements will not. This will permit the spatial, temporal and frequency control of the distribution of acoustic energy in the body. Techniques for altering the acoustic energy of the transducer elements are disclosed in U.S. Patent No. 5,520,612.

The following example is illustrative of the present invention.

EXAMPLE

The following example is illustrative of the manufacturing of a piezoelectric composite using the dice and fill method described above and use of the composite in an array to form a transducer in accordance with the present invention.

The fabrication processing of the transducer consisted of three main parts: (I) ceramic sample preparation and characterization, (II) dicing an filing method, and (III) array fabrication and final characterization. All the samples were processed from Morgan Matroc, Inc. PZT-5H powders. The as received powders contained approximately 3 weight percent polyvinyl alcohol (PVA) binder. To remove the initial binder from the powders, an initial binder burn out (BBO) cycle was performed in a furnace at 550°C for 3 hours. After this BBO step, the powders were mixed with exactly 6 weight percent of PVA binder solution (PVA/water ratio was 20/80) in a mortar and pestle and then sieved through a 70 mesh sieve.

Several pellets of 4,44 cm (1.75") in diameter were pressed uniaxially at $1,517.10^8$ Pa (22 ksi). An average weight of 26 g of PZT+binder powder was used for each pellet. The average green density was computed to be $4.58\pm0.02$ g/cc which is 58 percent of the theoretical density of PZT ($\rho_{th}$=7.86 g/cc). These pressed samples were subjected to a slow BBO cycle. An average of 1.15 weight percent loss was measured on the pellets after the BBO cycle. Sintering was carried out at 1285°C for 1 hour in a closed and sealed $Al_2O_3$ container. A mixture of $PbO+ZrO_2$ was used as a lead source to compensate for lead loss from the samples. In this step, the samples were placed on PZT-5H coarse powder (0.5-1.0 mm). An average of $0.3\pm0.06$ weight percent lead loss was found in the samples after sintering. The mean sintering density, by measuring the mass and dimensions of the sample, was found to be $7.45\pm0.01$ g/cc (approximately $94.7\pm0.2\%$ of the theoretical value).

The sintered samples were then polished with a dicing saw. Next, a 1200 SiC grit paper was used to smooth the surfaces. Silver paint was applied on one of the faces of the pellets and poled by the corona poling method. In this technique, the unelectroded top surface of the sample is exposed to a shower of positive ions while the bottom surface is grounded to a metallic plate placed on a hot plate. A potential of 25 kV was applied to the corona needles, ionizing the air between the tips of the needles and the metallic grounded plate which was 45 millimeters away from the needle tips. Poling was done for 15 minutes with the temperature being 70°C during the poling process. Then, the silver was removed with acetone and both faces of the samples were gold sputtered. The samples were left to age for 24 hours at room temperature with the gold electrodes short circuited, before physical and electrical characterization.

Capacitance ($C\rho$) and dissipation factor ($\tan\delta$) were measured at 1 kHz with a 1689M precision RLC Digibridge (GenRad Inc., Boston, MA). The relative dielectric constant $K$ of a sample of thickness $t$ was calculated by:

$$k = \frac{C_\rho t}{\varepsilon_o A}$$

where $\varepsilon_o$ is the permitivity of free space ($8.85\times10^{-12}$ F/m), and $A$ is the electroded area. The piezoelectric charge coefficient $d_{33}$ of the composites was measured at 100 Hz using a Berlincourt Piezo $d_{33}$-Meter (CPDT-3300-Chanel Product, Inc., Cleveland, OH). Two flat probes were used to test the composite and two round probes were used for ceramic samples.

The thickness and planar coupling coefficients were calculated by measuring the resonance and antiresonance frequencies with an Impedance/Gain-Phase Analyzer (4194A Hewlett-Packard Inc., Palo Alto, CA) and applying the following equations:

$$k_\rho^2 = \frac{f_\rho - f_s}{f_s}\left[\frac{R_1^2 - (1 - \sigma^2)}{1 + \sigma^2}\right]$$

$$k_t^2 \approx \frac{\pi}{2} \frac{f_s}{f_p} \tan\left(\frac{\pi}{2} \frac{f_p - f_s}{f_p}\right) \quad .$$

where $k_t$ and $k_p$ are thickness and planar coupling coefficients, $f_p$-$f_s$ is the parallel and series resonance frequencies difference, and the value of $R_l(\approx 2.05)$ is a function of Poisson's ratio for $\sigma = 0.3$. The average dielectric constant, dissipation factor, $d_{33}$, $k_t$ and $k_p$ coefficients of the pellet samples were $3021\pm36$, $0.0187\pm0.0005$, $608\pm5$ pC/N, 56% and 73% respectively.

The poled samples were then cut into a 2,54 x 2,54 cm$^2$ (1 x 1 in$^2$) plate and diced with a diamond-dicing saw into a modified 2-2 (or 2-(1-3)-2) connectivity pattern. The thickness of the ceramic wall ($p$), the spacing ($e$) between them and the connectivity were chosen to achieve the desired direction of flexibility and the volume faction of the final element. The volume fraction of the piezoelectric diced ceramic ($V^f_c$) is related to $p$ and $e$ by:

$$V^f_c = \frac{p}{p+e} \times \frac{p'}{p'+e'}$$

The primed terms indicate wall and spacing dimensions, cut in different perpendicular directions. Low viscosity hard Spurr polymer was used to refill the diced ceramics elements. The polymer was then removed from the ceramic, leaving a thin spurr film behind. Curing of this film at 70 °C for 12 hours was performed.

The composites were then arranged in a 4x3 array and back-filled with epoxy as follows: twelve different one-inch squared PZT/polymer composite elements were arranged in a rectangular 4 x 3 array, with 1-mm separation between elements. The thickness was chosen to obtain a characteristic resonance frequency of 1MHz when the volume fraction of the PZT in the composite was equal to 30%. The single composites were placed in a plastic dish (with same polarity up or down) with the specified 4 x 3 arrangement. The positive sides of all elements were glued to the plastic dish to prevent any displacement during the curing stage since the polymer may expand during caring which can change the original layout of elements.

The arrays were back-filled with Stycast 1365-65, de-aired, and cured. The cured array composites were polished to desired thickness corresponding to 1 MHz resonance frequency. Polishing was performed using a dicing saw (Kulicke & Soffa Industries Inc., Model 775 wafer saw, Horsham, PA). A 2 mm thick 340-mesh silicon carbide blade was used in this process. The blade swept the surface of array composite with 1-millimeter increment in x-y plane and 100 μm depth in z-direction. Since Stycast 1365-65 has higher Poisson's ratio than PZT ($\sigma\approx0.3$), using this small increments of depth was necessary to obtain a uniformly polished surface after each sweep.

The negative sides of arrays were gold sputtered to form common electrodes. The gold sputtering was accomplished using a dc-sputter coating (Polaron Instruments, SE Coating Unit E5100, Doylestown, PA) at room temperature and 20 mA dc-current under -808,64 mbar (-608 mmHg) pressure (with argon gas flow) for 4 minutes. Repoling is ordinarily not necessary in these diced composites arrays. Typical electromechanical properties of the PZT 2-(1-3)-2 array are set forth below in Table 1.

Table 1.

| Acoustic and electromechanical properties of 2-(1-3)-2 flexible array. | | | | | |
|---|---|---|---|---|---|
| K@1kHa | Tan δ@1KHz | $d_{33}$ (pC/N) | v (m/s) | Z (Mrayls) | $k_t$ (%) |
| 423±20 | 0.0240±0.0006 | 400±16 | 3280±23 | 9 | 63±1 |

Single element and three-element array composites were tested for flexibility using the previously described four-point bend test and withstood a 2mm deflection without failure.

Although the present invention has been described in preferred forms with a certain degree of particularity, many changes and variations are possible therein and will be apparent to those skilled in the art after reading the foregoing description. For example, while the foregoing description relates to medical applications, one skilled in the art would appreciate that the flexible composite transducer elements and large-area arrays of such transducer elements would also be used for medical diagnostic ultrasound applications and nondestructive testing such as, for example, nonmedical applications. As another example, instead of linear intersecting channels, spaces for receiving polymer can be provided in other configurations (e.g., pores, voids, arcuate channels, etc.) to achieve the desired volume of polymer loading. In such embodiments, a film of a first polymeric material would be applied in the spaces in the piezoelectric material and then the spaces would be filed with a second polymeric material in accordance with the previously described embodiments. It is therefore to be understood that the present invention may be practiced otherwise than as specifically described herein without departing from the spirit and scope thereof.

**Claims**

1. A piezoelectric composite transducer element (20) comprising islands (15) of piezoelectric material (10) separated by channels (12, 14) interconnecting each other,
   **characterized by**:
   a film (18) of a first polymeric material formed on the islands (15) of piezoelectric material (10) and a second polymeric material (19) for providing flexibility of the transducer element disposed over the film (18) and filling said channels (12, 14).

2. The transducer element (20) of Claim 1 wherein the piezoelectric material (10) is selected from the group consisting of PZT powder, ceramic, single-crystal relaxor ferroelectric, lead zirconate titanate $Pb(Zr,Ti)O_3$, lead metaniobate $Pb(Nb_2O_6)$, modified lead titanate $PbTi_3$, $(Pb,Ca)TiO_3$, $(Pb,Sm)TiO_3$, barium titanate $BaTiO_3$, PMN-PT$(1-x)Pb$ $(Mg_{1/3}Nb_{2/3})O_3$-$xPbTiO_3$, PZN-FT/BT $Pb(Zn_{1/3}Nb_{2/3})O_3$-$xPbTiO_3$-$BaTiO_3$, $(1-x)$ $Pb(Zn_{1/3}, Nb_{2/3})O_3$-$x$ $(yPbTiO_3$-$(1-y)PbZrO_3)$ and mixtures thereof.

3. The transducer element (20) of Claim 1 wherein the first polymeric material is selected from the group consisting of thermoplastics, thermosets, rubbers and mixtures thereof.

4. The transducer element (20) of Claim 1 wherein the first polymeric material is an epoxy.

5. The transducer element (20) of Claim 1 wherein the second polymeric material (19) is selected from the group consisting of thermoplastics, thermosets, rubbers and mixtures thereof.

6. The transducer element (20) of Claim 1 wherein the second polymeric material (19) is an epoxy.

7. The transducer element (20) of Claim 1 further comprising an electrode surface (38, 40) applied to and substantially coextensive with, opposite surfaces of the piezoelectric material (10) and a matching layer (16) applied to, and substantially coextensive with, one of the electrode surfaces (40).

8. The transducer element (20) of Claim 7 wherein the matching layer (16) comprises a second polymeric material and optionally a filler.

9. The transducer element (20) of Claim 8 wherein the second polymeric material is selected from the group consisting of thermoplastics, thermosets, rubbers and mixtures thereof.

10. The transducer element (20) of Claim 8 wherein the second polymeric material is an epoxy.

11. The transducer element (20) of Claim 8 wherein the matching layer (16) includes a filler selected from the group consisting of PZT, tungsten, alumina, silica glass, tungsten carbide and titanium.

12. The transducer element (20) of Claim 8 wherein the matching layer (16) includes glass powder as a filler.

13. The transducer element (20) of Claim 7 wherein the matching layer (16) has an acoustic impedance of from about 2.0 to about 7.0 MRayls.

14. The transducer element (20) of Claim 13 wherein the acoustic impedance of the matching layer is from about 3.0 to about 4.0 MRayls.

15. The transducer element (20) of Claim 1 wherein interconnecting channels (12, 14) are linear channels that intersect at an angle of between about 5° and 90°.

16. The transducer element (20) of Claim 15 wherein the channels (12, 14) intersect at an angle of about 30° to about 90°.

17. The transducer element (20) of Claim 1 having a flexibility of at least 2 mm.

18. The transducer element (20) of Claim 1 wherein the piezoelectric material (10) is PZT, the first and second polymeric material (19) are epoxies and the transducer element (20) has a flexibility of at least 2mm.

19. The transducer element (20) of any one of claims 1 to 18 being arranged with other piezoelectric composite transducer elements (20) so as to form a flexible piezoelectric transducer array, the piezoelectric composite transducer elements (20) being arranged in adjacent relation to define spaces (36) therebetween, the spaces (36) being filled with a third polymeric material;
one electrode surface (38) applied onto each transducer element (20) on one surface of the array and a second electrode surface (40) applied to, and substantially coextensive with, the other surface of the array; and,
a matching layer (16) applied to, and substantially coextensive with, the second electrode surface (40).

20. The transducer element (20) of claim 19 wherein the third polymeric material is selected from the group consisting of thermoplastics, thermosets, rubbers and mixtures thereof.

21. The transducer element (20) of claim 19 where in the third polymeric material is an epoxy.

22. The transducer element (20) of claim 19 wherein the matching layer (16) comprises a fourth polymeric material and optionally a filler.

23. The transducer element (20) of claim 22 wherein the fourth polymeric material is selected according to claim 9 or 10.

24. The transducer element (20) of any one of claims 19 to 22 wherein the transducer array is provided in accordance to any one of claims 11 to 16 and 18.

25. The transducer element (20) of claim 19 or 24 wherein the transducer array has a flexibility of at least about 2,0 mm.

26. A method for manufacturing a flexible piezoelectric transducer array comprising:

    a) forming a composite transducer element (20) according to any one of claims 1 to 18 by

        i) forming a first set of channels (12) parallel to one another within a piezoelectric material (10);
        ii) forming a second set of channels (14) within the piezoelectric material (10), the second set of channels (14) being parallel to one another and angularly oriented to the first set of channels (12);
        iii) forming a film (18) of a first polymeric material on the islands (15) of the piezoelectric material (10); and
        iv) covering the film (18) with a second polymeric material (19) in an amount sufficient to at least substantially fill the first and second sets of channels (12, 14);

    b) forming an array by positioning a plurality of the composite transducer elements (20) in adjacent relation to define spaces (36) therebetween;

    c) filling the spaces (36) between the plurality of positioned composite transducer elements (20) with a third polymeric material;

    d) applying one electrode surface (38) to each transducer element (20) on one surface of the array and a second electrode surface (40) applied to, and substantially coextensive with, the other surface of the array; and,

    e) applying a matching layer (16) applied to, and substantially coextensive with, the second electrode surface (40).

27. The method of Claim 26 wherein the piezoelectric material (10) is selected from the group consisting of PZT powder, ceramic, single-crystal relaxor ferroelectric, lead zirconate titanate $Pb(Zr,Ti)O_3$, lead metaniobate $Pb(Nb_2O_6)$, modified lead titanate $PbTi_3$, $(Pb,Ca)TiO_3$, $(Pb,Sm)TiO_3$, barium titanate $BaTiO_3$, PMN-PT$(1-x)Pb(Mg_{1/3}Nb_{2/3})O_3$-$xPbTiO_3$, PZN-PT/BT $Pb(Zn_{1/3}Nb_{2/3})O_3$-$xPbTiO_3$-$BaTiO_3$, $(1-x)$ $Pb(Zn_{1/3}, Nb_{2/3})O_3$-$x(yPbTiO_3$-$(1-y)PbZrO_3)$ and mixtures thereof.

28. The method of Claim 26 wherein the first polymeric material is selected from the group consisting of thermoplastics, thermosets, rubbers and mixtures thereof.

**29.** The method of Claim 26 wherein the first polymeric material is an epoxy.

**30.** The method of Claim 26 wherein the second polymeric material (19) is selected from the group consisting of thermoplastics, thermosets, rubbers and mixtures thereof.

**31.** The method of Claim 26, wherein the second polymeric material (19) is an epoxy.

**32.** The method of Claim 26 wherein the third polymeric material is selected from the group consisting of thermoplastics, thermosets, rubbers and mixtures thereof.

**33.** The method of Claim 26 wherein the third polymeric material is an epoxy.

**34.** The method of Claim 26 wherein the matching layer (16) comprises a fourth polymeric material and optionally a filler.

**35.** The method of Claim 34 wherein the fourth polymeric material is selected from the group consisting of thermoplastics, thermosets, rubbers and mixtures thereof.

**36.** The method of Claim 34, wherein the fourth polymeric material is an epoxy.

**37.** The method of Claim 34 wherein the matching layer (16) includes a filler selected from the group consisting of PZT, tungsten, alamina, silica glass, tungsten carbide and titanium.

**38.** The method of Claim 34 wherein the matching layer (16) includes glass powder as a filler.

**39.** The method of Claim 26 wherein the matching layer (16) has an acoustic impedance of from about 2.0 to about 7.0 MRayls.

**40.** The method of Claim 39 wherein the acoustic impedance of the matching layer (16) is from about 3.0 to about 4.0 MRayls.

**41.** The method of Claim 26 wherein the transducer (20) has a flexibility of at least about 2.0 mm.

**42.** The method of Claim 26 wherein the first set of channels (12) intersect the second set of channels (14) at an angle of between about 5° and about 90°.

**43.** The method of Claim 42 wherein the channels (12, 14) intersect at an angle of between about 30° and about 90°.

**44.** The method of Claim 26, wherein the piezoelectric material (10) is PZT, the first and second polymeric materials (19) are epoxies and the array has a PZT, the first and second polymeric materials (19) are epoxies and the array has a flexibility of at least 2mm.

**Patentansprüche**

**1.** Ein piezoelektrisches Verbundwandlerelement (20), das Inseln (15) aus piezoelektrischem Material (10), die durch einander verbindende Kanäle (12, 14) getrennt sind, beinhaltet,
**gekennzeichnet durch**:
einen Film (18) aus einem ersten polymeren Material, der auf den Inseln (15) aus piezoelektrischem Material (10) gebildet ist, und ein zweites polymeres Material (19), um Biegsamkeit des Wandlerelements bereitzustellen, das über dem Film (18) angeordnet ist und die Kanäle (12, 14) ausfüllt.

**2.** Wandlerelement (20) gemäß Anspruch 1, wobei das piezoelektrische Material (10) aus der Gruppe, die aus Bleizirkonattitanat-Pulver, Keramik, Einkristall-Relaxor-Ferroelektrika, Bleizirkonattitanat $Pb(Zr,Ti)O_3$, Bleimetaniobat $Pb(Nb_2O_6)$, modifiziertem Bleititanat $PbTi_3$, $(Pb,Ca)TiO_3$, $(Pb,Sm)TiO_3$, Bariumtitanat $BaTiO_3$, PMN-PT(1-x)Pb $(Mg_{1/3}Nb_{2/3})O_3xPbTiO_3$, PZN-PTIBT $Pb(Zn_{1/3}Nb_{2/3})O_3$-$XPbTiO_3$-$BaTiO_3$, (1-x) $Pb(Zn_{1/3}, Nb_{2/3})O_3$-x $(yPbTiO_3$-(1-y)$PbZrO_3)$ und Mischungen davon besteht, ausgewählt ist.

**3.** Wandlerelement (20) gemäß Anspruch 1, wobei das erste polymere Material aus der Gruppe, die aus Thermoplasten, warmausgehärteten Kunststoffen, Gummis und Mischungen davon besteht, ausgewählt ist.

4. Wandlerelement (20) gemäß Anspruch 1, wobei das erste polymere Material ein Epoxidharz ist.

5. Wandlerelement (20) gemäß Anspruch 1, wobei das zweite polymere Material (19) aus der Gruppe, die aus Thermoplasten, warmausgehärteten Kunststoffen, Gummis und Mischungen davon besteht, ausgewählt ist.

6. Wandlerelement (20) gemäß Anspruch 1, wobei das zweite polymere Material (19) ein Epoxidharz ist.

7. Wandlerelement (20) gemäß Anspruch 1, das ferner eine Elektrodenoberfläche (38, 40), die auf die gegenüberliegenden Oberflächen des piezoelektrischen Materials (10) aufgetragen ist und im Wesentlichen mit ihnen flächengleich ist, und eine passende Schicht (16), die auf eine der Elektrodenoberflächen (40) aufgetragen ist und im Wesentlichen mit ihr flächengleich ist, beinhaltet.

8. Wandlerelement (20) gemäß Anspruch 7, wobei die passende Schicht (16) ein zweites polymeres Material und wahlweise einen Füllstoff beinhaltet.

9. Wandlerelement (20) gemäß Anspruch 8, wobei das zweite polymere Material aus der Gruppe, die aus Thermoplasten, warmausgehärteten Kunststoffen, Gummis und Mischungen davon besteht, ausgewählt ist.

10. Wandlerelement (20) gemäß Anspruch 8, wobei das zweite polymere Material ein Epoxidharz ist.

11. Wandlerelement (20) gemäß Anspruch 8, wobei die passende Schicht (16) einen Füllstoff umfasst, der aus der Gruppe, die aus Bleizirkonattitanat, Wolfram, Tonerde, Hartglas, Wolframkarbid und Titan besteht, ausgewählt ist.

12. Wandlerelement (20) gemäß Anspruch 8, wobei die passende Schicht (16) Glaspulver als Füllstoff umfasst.

13. Wandlerelement (20) gemäß Anspruch 7, wobei die passende Schicht (16) eine akustische Impedanz von ungefähr 2,0 bis ungefähr 7,0 MRayl aufweist.

14. Wandlerelement (20) gemäß Anspruch 13, wobei die akustische Impedanz der passenden Schicht von ungefähr 3,0 bis ungefähr 4,0 MRayl beträgt.

15. Wandlerelement (20) gemäß Anspruch 1, wobei die verbindenden Kanäle (12, 14) lineare Kanäle sind, die sich in einem Winkel von zwischen ungefähr 5° und 90° schneiden.

16. Wandlerelement (20) gemäß Anspruch 15, wobei sich die Kanäle (12, 14) in einem Winkel von ungefähr 30° bis ungefähr 90° schneiden.

17. Wandlerelement (20) gemäß Anspruch 1, das eine Biegsamkeit von mindestens 2 mm aufweist.

18. Wandlerelement (20) gemäß Anspruch 1, wobei das piezoelektrische Material (10) Bleizirkonattitanat ist, das erste und das zweite polymere Material (19) Epoxidharze sind und das Wandlerelement (20) eine Biegsamkeit von mindestens 2 mm aufweist.

19. Wandlerelement (20) gemäß einem der Ansprüche 1 bis 18, das mit anderen piezoelektrischen Verbundwandlerelementen (20) so gruppiert ist, dass es eine biegsame piezoelektrische Wandleranordnung bildet, wobei die piezoelektrischen Verbundwandlerelemente (20) in angrenzender Beziehung gruppiert sind, um zwischen sich Räume (36) zu definieren, wobei die Räume (36) mit einem dritten polymeren Material gefüllt sind;
eine Elektrodenoberfläche (38), die auf jedes Wandlerelement (20) auf einer Oberfläche der Anordnung aufgetragen ist, und eine zweite Elektrodenoberfläche (40), die auf die andere Oberfläche der Anordnung aufgetragen ist und mit ihr flächengleich ist; und
eine passende Schicht (16), die auf die zweite Elektrodenoberfläche (40) aufgetragen ist und im Wesentlichen mit ihr flächengleich ist.

20. Wandlerelement (20) gemäß Anspruch 19, wobei das dritte polymere Material aus der Gruppe, die aus Thermoplasten, warmausgehärteten Kunststoffen, Gummis und Mischungen davon besteht, ausgewählt ist.

**21.** Wandlerelement (20) gemäß Anspruch 19, wobei das dritte polymere Material ein Epoxidharz ist.

**22.** Wandlerelement (20) gemäß Anspruch 19, wobei die passende Schicht (16) ein viertes polymeres Material und wahlweise einen Füllstoff beinhaltet.

**23.** Wandlerelement (20) gemäß Anspruch 22, wobei das vierte polymere Material gemäß Anspruch 9 oder 10 ausgewählt ist.

**24.** Wandlerelement (20) gemäß einem der Ansprüche 19 bis 22, wobei die Wandleranordnung gemäß einem der Ansprüche 11 bis 16 und 18 bereitgestellt ist.

**25.** Wandlerelement (20) gemäß Anspruch 19 oder 24, wobei die Wandleranordnung eine Biegsamkeit von mindestens ungefähr 2,0 mm aufweist.

**26.** Ein Verfahren zur Herstellung einer biegsamen piezoelektrischen Wandleranordnung, die Folgendes beinhaltet:

a) Bilden eines Verbundwandlerelements (20) gemäß einem der Ansprüche 1 bis 18 durch

i) Bilden eines ersten Satzes von Kanälen (12), die zueinander parallel sind, innerhalb eines piezoelektrischen Materials (10);

ii) Bilden eines zweiten Satzes von Kanälen (14) innerhalb des piezoelektrischen Materials (10), wobei die Kanäle (14) des zweiten Satzes parallel zueinander und in einem Winkel zu dem ersten Satz von Kanälen (12) orientiert sind;

iii) Bilden eines Films (18) aus einem ersten polymeren Material auf den Inseln (15) aus dem piezoelektrischen Material (10); und

iv) Bedecken des Films (18) mit einem zweiten polymeren Material (19) in einer Menge, die ausreicht, um den ersten und den zweiten Satz von Kanälen (12,14) mindestens im Wesentlichen zu füllen;

b) Bilden einer Anordnung durch das Positionieren einer Vielzahl der Verbundwandlerelemente (20) in angrenzender Beziehung, um zwischen ihnen Räume (36) zu definieren;

c) Füllen der Räume (36) zwischen der Vielzahl von positionierten Verbundwandlerelementen (20) mit einem dritten polymeren Material;

d) Auftragen einer Elektrodenoberfläche (38) auf jedes Wandlerelement (20) auf einer Oberfläche der Anordnung und einer zweiten Elektrodenoberfläche (40), die auf die andere Oberfläche der Anordnung aufgetragen wird und mit ihr flächengleich ist; und

e) Auftragen einer passenden Schicht (16), die auf die zweite Elektrodenoberfläche (40) aufgetragen wird und im Wesentlichen mit ihr flächengleich ist.

**27.** Verfahren gemäß Anspruch 26, wobei das piezoelektrische Material (10) aus der Gruppe, die aus Bleizirkonattitanat-Pulver, Keramik, Einkristall-Relaxor-Ferroelektrika, Bleizirkonattitanat $Pb(Zr,Ti)O_3$, Bleimetaniobat $Pb(Nb_2O_6)$, modifiziertem Bleititanat $PbTi_3$, $(Pb,Ca)TiO_3$, $(Pb,Sm)TiO_3$, Bariumtitanat $BaTiO_3$, PMN-PT(1-x)Pb $(Mg_{1/3}Nb_{2/3})O_3$-x$PbTiO_3$, PZN-PT/BT $Pb(Zn_{1/3}Nb_{2/3})O_3$-x$PbTiO_3$-$BaTiO_3$, (1-x) $Pb(Zn_{1/3}, Nb_{2/3})O_3$-x $(yPbTiO_3$-(1-y)$PbZrO_3)$ und Mischungen davon besteht, ausgewählt ist.

**28.** Verfahren gemäß Anspruch 26, wobei das erste polymere Material aus der Gruppe, die aus Thermoplasten, warmausgehärteten Kunststoffen, Gummis und Mischungen davon besteht, ausgewählt ist.

**29.** Verfahren gemäß Anspruch 26, wobei das erste polymere Material ein Epoxidharz ist.

**30.** Verfahren gemäß Anspruch 26, wobei das zweite polymere Material (19) aus der Gruppe, die aus Thermoplasten, warmausgehärteten Kunststoffen, Gummis und Mischungen davon besteht, ausgewählt ist.

**31.** Verfahren gemäß Anspruch 26, wobei das zweite polymere Material (19) ein Epoxidharz ist.

**32.** Verfahren gemäß Anspruch 26, wobei das dritte polymere Material aus der Gruppe, die aus Thermoplasten, warmausgehärteten Kunststoffen, Gummis und Mischungen davon besteht, ausgewählt ist.

**33.** Verfahren gemäß Anspruch 26, wobei das dritte polymere Material ein Epoxidharz ist.

**34.** Verfahren gemäß Anspruch 26, wobei die passende Schicht (16) ein viertes polymeres Material und wahlweise einen Füllstoff beinhaltet.

**35.** Verfahren gemäß Anspruch 34, wobei das vierte polymere Material aus der Gruppe, die aus Thermoplasten, warmausgehärteten Kunststoffen, Gummis und Mischungen davon besteht, ausgewählt ist.

**36.** Verfahren gemäß Anspruch 34, wobei das vierte polymere Material ein Epoxidharz ist.

**37.** Verfahren gemäß Anspruch 34, wobei die passende Schicht (16) einen Füllstoff umfasst, der aus der Gruppe, die aus Bleizirkonattitanat, Wolfram, Tonerde, Hartglas, Wolframkarbid und Titan besteht, ausgewählt ist.

**38.** Verfahren gemäß Anspruch 34, wobei die passende Schicht (16) Glaspulver als Füllstoff umfasst.

**39.** Verfahren gemäß Anspruch 26, wobei die passende Schicht (16) eine akustische Impedanz von ungefähr 2,0 bis ungefähr 7,0 MRayl aufweist.

**40.** Verfahren gemäß Anspruch 39, wobei die akustische Impedanz der passenden Schicht (16) ungefähr 3,0 bis ungefähr 4,0 MRayl beträgt.

**41.** Verfahren gemäß Anspruch 26, wobei der Wandler (20) eine Biegsamkeit von mindestens ungefähr 2,0 mm aufweist.

**42.** Verfahren gemäß Anspruch 26, wobei der erste Satz von Kanälen (12) den zweiten Satz von Kanälen (14) in einem Winkel von zwischen ungefähr 5° und ungefähr 90° schneidet.

**43.** Verfahren gemäß Anspruch 42, wobei sich die Kanäle (12,14) in einem Winkel von zwischen ungefähr 30° und ungefähr 90° schneiden.

**44.** Verfahren gemäß Anspruch 26, wobei das piezoelektrische Material (10) Bleizirkonattitanat ist, das erste und das zweite polymere Material (19) Epoxidharze sind und die Anordnung eine Biegsamkeit von mindestens 2 mm aufweist.

**Revendications**

**1.** Un élément transducteur composite piézoélectrique (20) comportant des îlots (15) de matériau piézoélectrique (10) séparés par des canaux (12, 14) raccordés entre eux,
**caractérisé par** :
un film (18) en un premier matériau polymère formé sur les îlots (15) de matériau piézoélectrique (10) et un deuxième matériau polymère (19) destiné à rendre flexible l'élément transducteur disposé sur le film (18) et à remplir lesdits canaux (12, 14).

**2.** L'élément transducteur (20) de la revendication 1 dans lequel le matériau piézoélectrique (10) est sélectionné dans le groupe consistant en poudre PZT, céramique, ferroélectrique relaxeur monocristallin, titanate de zirconium de plomb $Pb(Zr, Ti)O_3$, métanobiate de plomb $Pb(Nb_2O_6)$, titanate de plomb modifié $PbTi_3$, $(Pb, Ca)TiO_3$, $(Pb, Sm)TiO_3$, titanate de barium $BaTiO_3$, PMN-PT$(1-x)Pb(Mg_{1/3}Nb_{2/3})O_3$-$xPbTiO_3$, PZN-PTIBT $Pb(Zn_{1/3}Nb_{2/3})O_3$-$xPbTiO_3$-$BaTiO_3$, $(1-x) Pb(Zn_{1/3}, Nb_{2/3})O_3$-$x(yPbTiO_3$-$(1-y)PbZrO_3)$ et mélanges de ceux-ci.

**3.** L'élément transducteur (20) de la revendication 1 dans lequel le premier matériau polymère est sélectionné dans le groupe consistant en thermoplastiques, thermodurcis, caoutchoucs et mélanges de ceux-ci.

**4.** L'élément transducteur (20) de la revendication 1 dans lequel le premier matériau polymère est un époxy.

**5.** L'élément transducteur (20) de la revendication 1 dans lequel le deuxième matériau polymère (19) est sélectionné dans le groupe consistant en thermoplastiques, thermodurcis, caoutchoucs et mélanges de ceux-ci.

**6.** L'élément transducteur (20) de la revendication 1 dans lequel le deuxième matériau polymère (19) est un époxy.

**7.** L'élément transducteur (20) de la revendication 1 comportant de plus une surface d'électrode (38, 40) appliquée sur des surfaces opposées du matériau piézoélectrique (10) et substantiellement de même étendue que celles-ci, et une couche assortie (16) appliquée sur une des surfaces d'électrodes (40) et substantiellement de même étendue que celle-ci.

**8.** L'élément transducteur (20) de la revendication 7 dans lequel la couche assortie (16) comporte un deuxième matériau polymère et facultativement une matière de remplissage.

**9.** L'élément transducteur (20) de la revendication 8 dans lequel le deuxième matériau polymère est sélectionné dans le groupe consistant en thermoplastiques, thermodurcis, caoutchoucs et mélanges de ceux-ci.

**10.** L'élément transducteur (20) de la revendication 8 dans lequel le deuxième matériau polymère est un époxy.

**11.** L'élément transducteur (20) de la revendication 8 dans lequel la couche assortie (16) comprend une matière de remplissage sélectionnée dans le groupe consistant en PZT, tungstène, alumine, verre de silice, carbure de tungstène et titane.

**12.** L'élément transducteur (20) de la revendication 8 dans lequel la couche assortie (16) comprend de la poudre de verre comme matière de remplissage.

**13.** L'élément transducteur (20) de la revendication 7 dans lequel la couche assortie (16) a une impédance acoustique allant d'environ 2,0 à environ 7,0 MRayl.

**14.** L'élément transducteur (20) de la revendication 13 dans lequel l'impédance acoustique de la couche assortie est d'environ 3,0 à environ 4,0 MRayl.

**15.** L'élément transducteur (20) de la revendication 1 dans lequel les canaux de raccordement (12, 14) sont des canaux linéaires qui s'entrecoupent à un angle compris entre environ 5° et 90°.

**16.** L'élément transducteur (20) de la revendication 15 dans lequel les canaux (12, 14) s'entrecoupent à un angle allant d'environ 30° à environ 90°.

**17.** L'élément transducteur (20) de la revendication 1 ayant une flexibilité d'au moins 2 mm.

**18.** L'élément transducteur (20) de la revendication 1 dans lequel le matériau piézoélectrique (10) est le PZT, le premier et le deuxième matériau polymère (19) sont des époxy et l'élément transducteur (20) a une flexibilité d'au moins 2 mm.

**19.** L'élément transducteur (20) de n'importe laquelle des revendications 1 à 18 arrangé avec d'autres éléments transducteurs composites piézoélectriques (20) de manière à former un ensemble de transducteurs piézoélectriques flexibles, les éléments transducteurs composites piézoélectriques (20) étant arrangés en relation adjacente pour définir des espaces (36) entre eux, les espaces (36) étant remplis d'un troisième matériau polymère ;
une surface d'électrode (38) appliquée sur chaque élément transducteur (20) sur une surface de l'ensemble et une deuxième surface d'électrode (40) appliquée sur l'autre surface de l'ensemble, et substantiellement de même étendue que celle-ci ; et,
une couche assortie (16) appliquée sur la deuxième surface d'électrode (40), et substantiellement de même étendue que celle-ci.

**20.** L'élément transducteur (20) de la revendication 19 dans lequel le troisième matériau polymère est sélectionné dans le groupe consistant en thermoplastiques, thermodurcis, caoutchoucs et mélanges de ceux-ci.

**21.** L'élément transducteur (20) de la revendication 19 dans lequel le troisième matériau polymère est un époxy.

**22.** L'élément transducteur (20) de la revendication 19 dans lequel la couche assortie (16) comporte un quatrième matériau polymère et facultativement une matière de remplissage.

**23.** L'élément transducteur (20) de la revendication 22 dans lequel le quatrième matériau polymère est sélectionné selon la revendication 9 ou la revendication 10.

**24.** L'élément transducteur (20) de n'importe laquelle des revendications 19 à 22 dans lequel l'ensemble de transducteurs est fourni conformément à n'importe laquelle des revendications 11 à 16 et 18.

**25.** L'élément transducteur (20) de la revendication 19 ou la revendication 24 dans lequel l'ensemble de transducteurs a une flexibilité d'au moins 2,0 mm.

**26.** Un procédé de fabrication d'un ensemble de transducteurs piézoélectriques flexible comportant :

a) la formation d'un élément transducteur composite (20) selon n'importe laquelle des revendications 1 à 18 par

i) la formation d'un premier jeu de canaux (12) parallèles les uns aux autres à l'intérieur d'un matériau piézoélectrique (10) ;

ii) la formation d'un deuxième jeu de canaux (14) à l'intérieur du matériau piézoélectrique (10), les canaux du deuxième jeu de canaux (14) étant parallèles les uns aux autres et orientés angulairement au premier jeu de canaux (12) ;

iii) la formation d'un film (18) d'un premier matériau polymère sur les îlots (15) du matériau piézoélectrique (10) ; et

iv) la couverture du film (18) avec un deuxième matériau polymère (19) dans une quantité suffisante pour au moins substantiellement remplir les premier et deuxième jeux de canaux (12, 14) ;

b) la formation d'un ensemble en positionnant une pluralité des éléments transducteurs composites (20) en relation adjacente pour définir des espaces (36) entre eux ;

c) le remplissage des espaces (36) entre la pluralité d'éléments transducteurs composites positionnés (20) avec un troisième matériau polymère ;

d) l'application d'une surface d'électrode (38) sur chaque élément transducteur (20) sur une surface de l'ensemble et une deuxième surface d'électrode (40) appliquée sur l'autre surface de l'ensemble, et substantiellement de même étendue que cette-ci ; et,

e) l'application d'une couche assortie (16) appliquée sur la deuxième surface d'électrode (40), et substantiellement de même étendue que celle-ci.

**27.** Le procédé de la revendication 26 dans lequel le matériau piézoélectrique (10) est sélectionné dans le groupe consistant en poudre PZT, céramique, ferroélectrique relaxeur monocristallin, titanate de zirconium de plomb Pb $(Zr, Ti)O_3$, métanobiate de plomb $Pb(Nb_2O_6)$, titanate de plomb modifié $PbTi_3$, $(Pb, Ca)TiO_3$, $(Pb, Sm)TiO_3$, titanate de barium $BaTiO_3$, PMN-PT $(1-x)Pb(Mg_{1/3}Nb_{2/3})O_3$-$xPbTiO_3$, PZN-PTIBT $Pb(Zn_{1/3}Nb_{2/3})O_3$-$xPbTiO_3$- $BaTiO_3$, $(1-x) Pb(Zn_{1/3}, Nb_{2/3})O_3$-$x(yPbTiO_3$-$(1-y)PbZrO_3)$ et mélanges de ceux-ci.

**28.** Le procédé de la revendication 26 dans lequel le premier matériau polymère est sélectionné dans le groupe consistant en thermoplastiques, thermodurcis, caoutchoucs et mélanges de ceux-ci.

**29.** Le procédé de la revendication 26 dans lequel le premier matériau polymère est un époxy.

**30.** Le procédé de la revendication 26 dans lequel le deuxième matériau polymère (19) est sélectionné dans le groupe consistant en thermoplastiques, thermodurcis, caoutchoucs et mélanges de ceux-ci.

**31.** Le procédé de la revendication 26 dans lequel le deuxième matériau polymère (19) est un époxy.

**32.** Le procédé de la revendication 26 dans lequel le troisième matériau polymère est sélectionné dans le groupe consistant en thermoplastiques, thermodurcis, caoutchoucs et mélanges de ceux-ci.

**33.** Le procédé de la revendication 26 dans lequel le troisième matériau polymère est un époxy.

**34.** Le procédé de la revendication 26 dans lequel la couche assortie (16) comporte un quatrième matériau polymère et facultativement une matière de remplissage.

**35.** Le procédé de la revendication 34 dans lequel le quatrième matériau polymère est sélectionné dans le groupe consistant en thermoplastiques, thermodurcis, caoutchoucs et mélanges de ceux-ci.

**36.** Le procédé de la revendication 34 dans lequel le quatrième matériau polymère est un époxy.

**37.** Le procédé de la revendication 34 dans lequel la couche assortie (16) comprend une matière de remplissage sélectionnée dans le groupe consistant en PZT, tungstène, alumine, verre de silice, carbure de tungstène et titane.

**38.** Le procédé de la revendication 34 dans lequel la couche assortie (16) comprend de la poudre de verre comme matière de remplissage.

**39.** Le procédé de la revendication 26 dans lequel la couche assortie (16) a une impédance acoustique allant d'environ 2,0 à environ 7,0 MRayl.

**40.** Le procédé de la revendication 39 dans lequel l'impédance acoustique de la couche assortie (16) est d'environ 3,0 à environ 4,0 MRayl.

**41.** Le procédé de la revendication 26 dans lequel le transducteur (20) a une flexibilité d'au moins 2,0 mm.

**42.** Le procédé de la revendication 26 dans lequel le premier jeu de canaux (12) entrecoupe le deuxième jeu de canaux (14) à un angle compris entre environ 5° et environ 90°.

**43.** Le procédé de la revendication 42 dans lequel les canaux (12, 14) s'entrecoupent à un angle compris entre environ 30° et environ 90°.

**44.** Le procédé de la revendication 26 dans lequel le matériau piézoélectrique (10) est du PZT, les premier et deuxième matériaux polymères (19) sont des époxy et l'ensemble a une flexibilité d'au moins 2 mm.

FIGURE 1A

FIGURE 1B

FIGURE 1C

FIGURE 2